# EUROPEAN PATENT APPLICATION

(11) **EP 4 796 155 A2**
(43) Date of publication of application: **26.08.2026**
(21) Application number: 26189767.2
(22) Date of filing: 29.05.2021
(51) Int. Cl.: A61M 16/10

(54) **DEVICES AND RELATED METHODS FOR VENTILATION**

(30) Priority: 24.06.2020 US 202063043127 P; 04.02.2021 US 202163145689 P
(62) Document number(s) of the earlier application(s) in accordance with Art. 76 EPC: 21828271.3 / 4 171 693
(71) Applicant: FluidIQ INC., Winthrop, MA 02150 (US); Lunday, Jake, Highland Village, TX 75077 (US); Froelke, Brian, St. Louis, MO 63129 (US); Stevenson, Duncan, 2055 Johannesburg (ZA); Walsh, Brian, Lynchburg, VA 24504 (US); Mendoza, Artemio, Katy, TX 77450 (US)
(72) Inventor: LUNDAY, Jake, Highland Village, Texas, 75077 (US); FROELKE, Brian, St Louis, Missouri 63129 (US); STEVENSON, Duncan, 2055 Johannesburg (ZA); WALSH, Brian, Lynchburg, Virginia 24504 (US); MENDOZA, Artemio, Katy, Texas 77450 (US); JUNG, Chris, deceased (US)
(74) Representative: Mohun, Stephen John

(57) **Abstract**

Provided herein are devices and methods that relate to ventilation and respiration. In one embodiment, a ventilator device comprising a fluidic amplifier with one or more coaxially aligned components, where there are no internal moving components. In another embodiment, a device and related methods for treating a patient who needs ventilation such as after infection by the coronavirus Covid-19.

## Description

### FIELD OF THE INVENTION

The present disclosure relates to the field of health care, and specifically ventilation.

### BACKGROUND OF THE DISCLOSURE

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

Mechanical ventilation can be a vital component of critical care services for patients, and when mechanical ventilation is not deployed properly or safely, it can have disastrous effects. However, the challenges responding to the recent global pandemic caused by the coronavirus COVID-19 has highlighted a great need for ventilators that may be both effective and reliable, as well as designed to be quickly manufactured and employed where needed. During the COVID-19 pandemic, there was a major shortage of mechanical ventilators due to an unexpected surge of patients suffering from life-threating respiratory failure. There was an immediate need and short supply for effective, inexpensive and simple to use ventilators and resuscitators. For example, in the U.S., there was an immediate and great need for effective, inexpensive and simple to use ventilators and resuscitators to help bridge the gap during the COVID-19 onset of respiratory failure outside of the ICU setting (EMS, emergency rooms, chronic recovery units) and inside the ICU when full featured ventilators were in short supply. Or, for example, such a ventilator or resuscitator may also be needed in future regional epidemic episodes or in under-resourced environments. Thus, there is a need in the art for novel and effective ventilation devices and related components.

### BRIEF SUMMARY OF THE INVENTION

Various embodiments include a device comprising a fluidic amplifier with a plurality of coaxially aligned components. In one embodiment, the fluidic amplifier comprises a Channel Depth for controlling pressure and/or volume of gas moving through the device. In another embodiment, the Channel Depth influences Respiratory Rate for a user. In another embodiment, the Channel Depth comprises a tear drop shaped channel for stabilizing incoming oxygen gas. In another embodiment, the Channel Depth comprises an inspiration phase channel and/or an expiration phase channel. In another embodiment, the fluidic amplifier comprises a nozzle width for controlling velocity of gas moving through the device. In another embodiment, the fluidic amplifier ventilates a user by fluidics and pressure capacitance. In another embodiment, the fluidic amplifier provides a Respiratory Rate of 2 to 200 bpm. In another embodiment, the fluidic amplifier provides a Respiratory Rate of 10 to 40 bpm. In another embodiment, the fluidic amplifier provides a Respiratory Rate of 15 to 35 bpm. In another embodiment, the fluidic amplifier is color coded to correlate with a desired pressure. In another embodiment, the fluidic amplifier utilizes internal geometry and gas flow rate to provide a desired Peak Inspiratory Pressure (PIP), Positive End Expiratory Pressure (PEEP) and Inhale to Exhale Ratio (IE) for a user. In another embodiment, the fluidic amplifier is connected to a gas source. In another embodiment, the fluidic amplifier is connected to the gas source by means of an oxygen tubing. In another embodiment, the fluidic amplifier comprises a barb and/or threaded connector that connects to the oxygen tubing. In another embodiment, the plurality of coaxially aligned components include a Fluid Inlet, Nozzle, Biased Port Attachment Surface, Non-biased Port-Attachment Surface, Exhaust, Splitter, Outlet, Channel Depth, and/or Aero Offset. In another embodiment, the fluidic amplifier utilizes laminar air flow design. In another embodiment, the fluidic amplifier is adapted to provide emergency mechanical ventilation. In another embodiment, the device is part of an overall treatment regimen for infection by coronavirus Covid-19. In another embodiment, the device is disposable. In another embodiment, the plurality of coaxially aligned components are modular in design. In another embodiment, the device may be modified and/or adjusted by one or more geometries to achieve a desired set of parameters. In another embodiment, the device may be modified to achieve a desired set of parameters by one or more of the following geometries: width of Nozzle, depth of Channel, radius of Biased Port Attachment Surface, shape of non-biased outlet shape, divergence angle between biased channel and non-biased channel, and Aero Offset between Nozzle exit and start of radius. In another embodiment, the device is one of a discrete set that may be of different size and/or labeled to identify which device best meets the needs of a patient. In another embodiment, needs of the patient include measurement of levels of Peak Inspiratory Pressure (PIP), Positive End Expiratory Pressure (PEEP), and/or Respiratory Rate (RR). In another embodiment, the fluidic amplifier is integrated with a flow generating device. In another embodiment, the fluidic amplifier is connected to and/or incorporated into a Bag Valve Mask (BVM). In another embodiment, the fluidic amplifier provides a PIP of between 5 and 50 cmH2O. In another embodiment, the fluidic amplifier provides a PEEP of between 2 and 30 cmH2O. In another embodiment, the fluidic amplifier utilizes turbulent air flow design.

Other embodiments include a kit, comprising an apparatus comprising a fluidic amplifier adapted for ventilation of a subject. In another embodiment, the fluidic amplifier comprises one or more coaxially aligned components. In another embodiment, the fluidic amplifier comprises a fluid inlet and an outlet. In another embodiment, breathing gas is supplied to the fluid inlet. In another embodiment, the fluid inlet has a barbed fitting. In another embodiment, the fluidic amplifier is connected to a gas source by means of oxygen tubing. In another embodiment, flow rate of the gas source may be controlled by an upstream flow control valve. In another embodiment, the fluidic amplifier comprises a Channel Depth for controlling volume and/or pressure of gas moving through the device. In another embodiment, the Channel Depth comprises a tear drop shaped channel for stabilizing incoming oxygen gas. In another embodiment, the Channel Depth comprises an inspiration phase channel and/or an expiration phase channel. In another embodiment, the fluidic amplifier comprises a nozzle width for controlling velocity of gas moving through the device. In another embodiment, the fluidic amplifier ventilates a user by means of a fluidics and pressure capacitance mechanism. In another embodiment, the fluidic amplifier provides a Respiratory Rate (RR) of 5 to 500 bpm. In another embodiment, the kit comprises a low pressure fluidic amplifier, medium pressure fluidic amplifier, and/or a high pressure fluidic amplifier. In another embodiment, the fluidic amplifier with a RR between 10-19 bpm, a fluidic amplifier with a RR between 20-27 bpm, and/or a fluidic amplifier with a RR between 28-50 bpm. In another embodiment, one or more color coded fluidic amplifiers as part of an overall protocol for treating a severe lung condition in a patient. In another embodiment, the apparatus is operably linked to the subject for inspiration and expiration phases of ventilation. In another embodiment, comprising a Pressure Relief Valve (PRV), a Pressure Indicator, an Anti-Asphyxiation Valve, a Filter and/or an Oxygen Tube. In another embodiment, an ASV, manometer, and/or pressure limiter. In another embodiment, comprising a low pressure fluidic amplifier, a medium pressure fluidic amplifier, and a high pressure fluidic amplifier. In another embodiment, the apparatus provides pressure cycled mechanical ventilation.

Other embodiments include a method of treating a patient with an adverse condition, comprising providing a ventilation device comprising a fluidic amplifier with one or more coaxially aligned components, and treating the patient by ventilation. In another embodiment, the fluidic amplifier comprises one or more of the following components operably linked: Fluid Inlet, Nozzle, Biased Port Attachment Surface, Non-biased Port-Attachment Surface, Exhaust, Splitter, Outlet, Channel Depth, and Aero Offset. In another embodiment, the adverse condition is respiratory related. In another embodiment, the adverse condition is infection by coronavirus Covid-19. In another embodiment, the ventilation device comprises one or more color coded fluidic amplifiers with different pressure strength. In another embodiment, treatment is part of an overall protocol comprising a Green color coded fluidic amplifier of a Respiratory Rate (RR) strength between 15-18 bpm, a Yellow color coded fluidic amplifier of a RR strength between 19-26 bpm, and/or a Red color coded fluidic amplifier of a RR strength between 27-30 bpm. In another embodiment, the fluidic amplifier comprises one or more internal channels. In another embodiment, the one or more internal channels are visible to a user when viewed from outside of the device. In another embodiment, the ventilation device is stored digitally for mass production on demand during time of need. In another embodiment, the ventilation device is sent and stored digitally to enable custom manufacturing for use on a specific patient and/or patient population. In another embodiment, the ventilation device is printed at or near the point of use to treat a specific patient and/or patient population. In another embodiment, the fluidic amplifier is a monostable design. In another embodiment, the fluidic amplifier is a bistable design. In another embodiment, the adverse condition is sleep apnea. In another embodiment, the fluidic amplifier provides continuous positive airway pressure (CPAP). In another embodiment, the fluidic amplifier provides a Peak Inspiratory Pressure (PIP) of 9 cmH20, a Positive End Expiratory Pressure (PEEP) of 7 cmH2O, and/or a CPAP of 8 cmH2O. In another embodiment, the fluidic amplifier provides pressure cycled mechanical ventilation. In another embodiment, the treatment is for a mammal.

Other features and advantages of the invention will become apparent from the following detailed description, taken in conjunction with the accompanying drawings, which illustrate, by way of example, various embodiments of the invention.

### DESCRIPTION OF THE DRAWINGS

Exemplary embodiments are illustrated in referenced figures. It is intended that the embodiments and figures disclosed herein are to be considered illustrative rather than restrictive.
Figure 1 depicts, in accordance with various embodiments herein, an example of a fluidic amplifier device. As referenced in Figure 1, a fluidic amplifier with the following components: Fluid Inlet **101,** Nozzle **102,** Biased Port Attachment Surface **103,** Non-biased Port-Attachment Surface **104,** Exhaust **105,** Splitter **106,** Outlet **107,** Channel **108,** and Aero Offset **109.** In one embodiment, the device is a coaxial fluidic oscillator functioning as a mechanical ventilator.
Figure 2 depicts, in accordance with various embodiments herein, a flow chart describing an example of a protocol of a process of ventilation and evaluation for a patient using one or more devices comprising a fluidic amplifier with one or more coaxially aligned components. As further described herein, the protocol may start with the use of a Yellow color coded **111** fluidic amplifier, and then potentially change to other color coded fluidic amplifiers depending on whether or not it is ascertained that there is adequate ventilation to the patient.

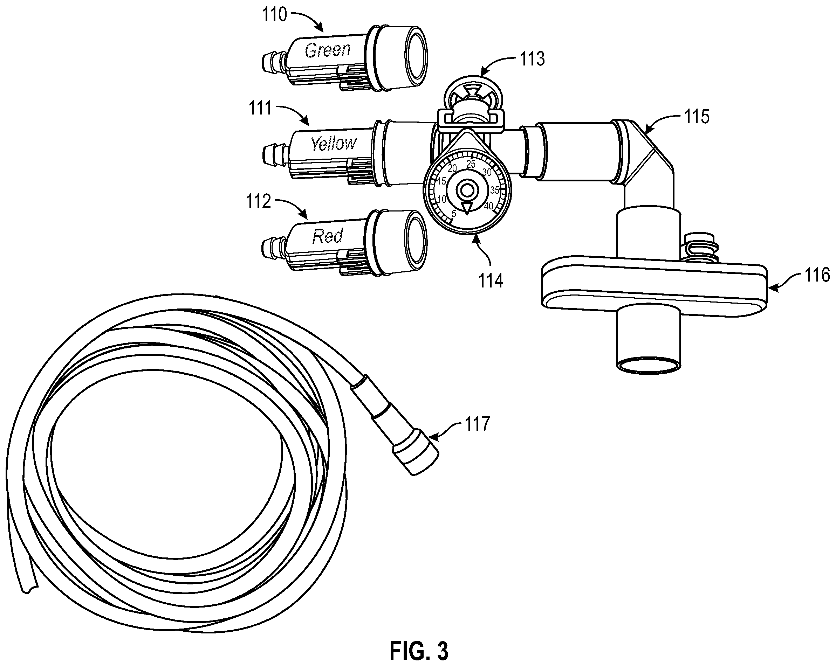

Figure 3 depicts, in accordance with an embodiment herein, an example of a single use, constant flow, pressure cycled ventilator designed to provide emergency mechanical ventilation. There are three examples of color coded fluidic amplifiers depicted, including Green color coded **110,** Yellow color coded **111,** and Red color coded **112.** The Yellow color coded **111** fluidic amplifier is depicted as inserted into a device for ventilation; however, as further described herein, each of the various color coded fluidic amplifiers could potentially be chosen and inserted for use in a ventilation device depending on desired ventilation parameters. In accordance with various embodiments herein, a Pressure Relief Valve **113,** Pressure Indicator **114,** Anti-Asphyxiation Valve **115,** and/or Filter **116** may also be incorporated. In one embodiment, the Filter **116** is a Heat and Moisture Exchange HEPA. In another embodiment, the Pressure Indicator **114** has a visual indicator for the user. In accordance with another embodiment, a kit comprising one or more fluidic amplifiers and Tubing **117.**

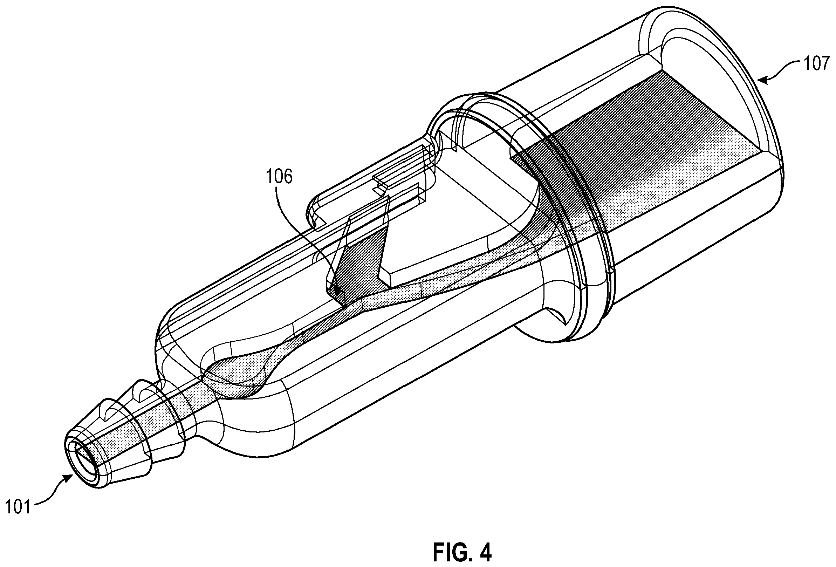

Figure 4 depicts, in accordance with various embodiments herein, the inspiratory phase of a fluidic amplifier.

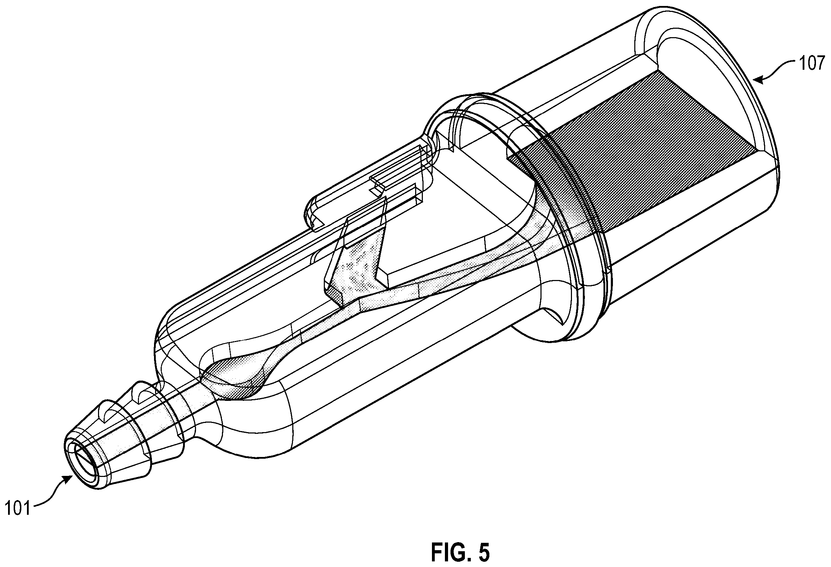

Figure 5 depicts, in accordance with various embodiments herein, the expiratory phase of a fluidic amplifier.

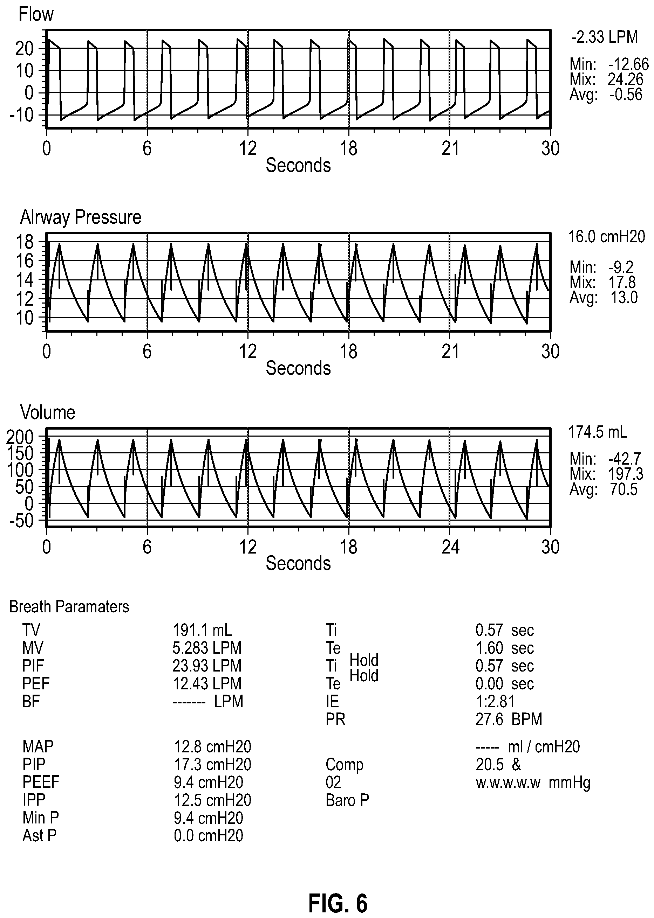

Figure 6 depicts, in accordance with various embodiments herein, some results from performance testing. The figure contains waveforms showing results of a fluidic amplifier in operation. In this instance, the waveforms are for a Red color coded fluidic amplifier that has been installed.

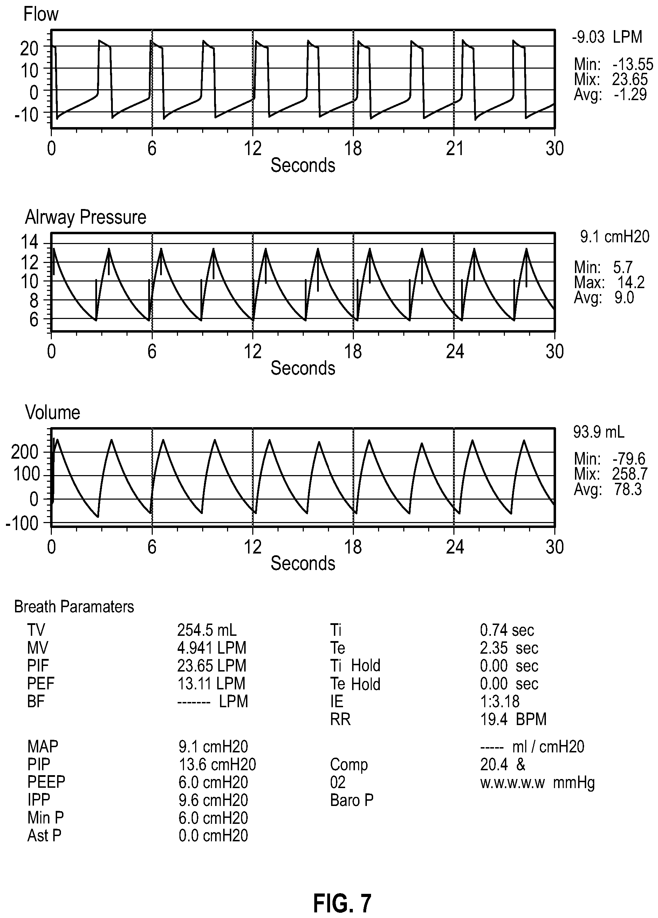

Figure 7 depicts, in accordance with various embodiments herein, some results from performance testing. The figure contains waveforms showing results of a fluidic amplifier in operation. In this instance, the waveforms are for a Yellow color coded fluidic amplifier that has been installed.

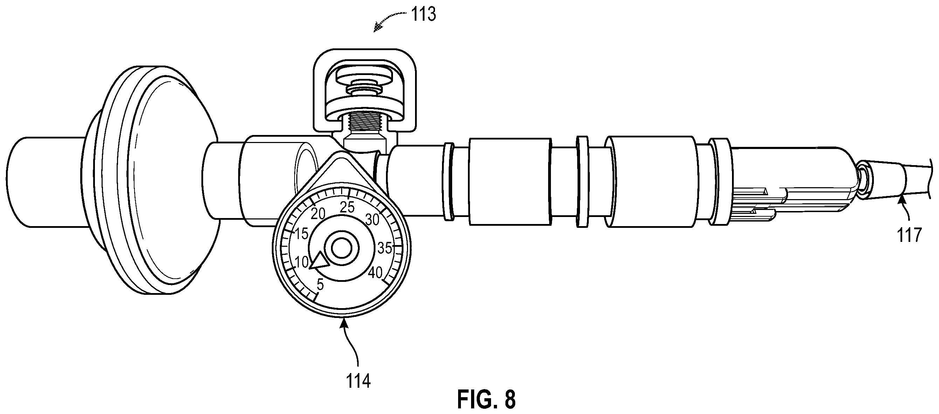

Figure 8 depicts, in accordance with various embodiments herein, ventilator and valves reconnected into a test lung.

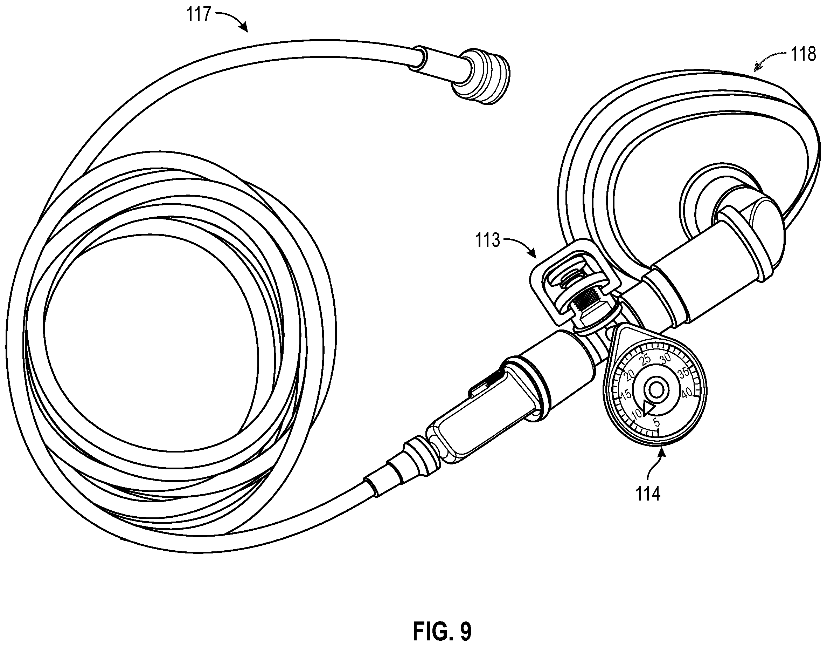

Figure 9 depicts, in accordance with various embodiments herein, an example of a fluidic amplifier that is attached to a face mask **118** that may be used by a patient for breathing.

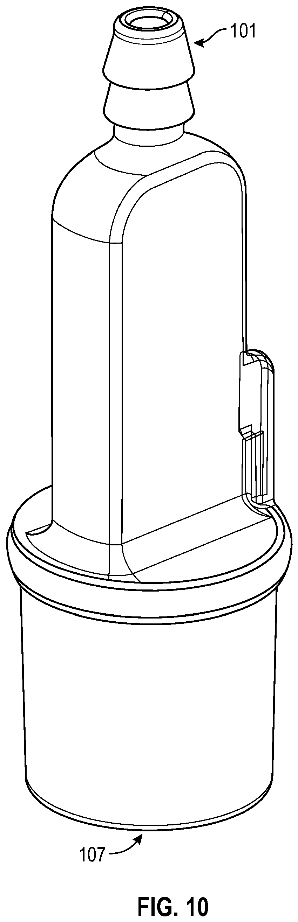

Figure 10 depicts, in accordance with various embodiments herein, an example of a fluidic amplifier.

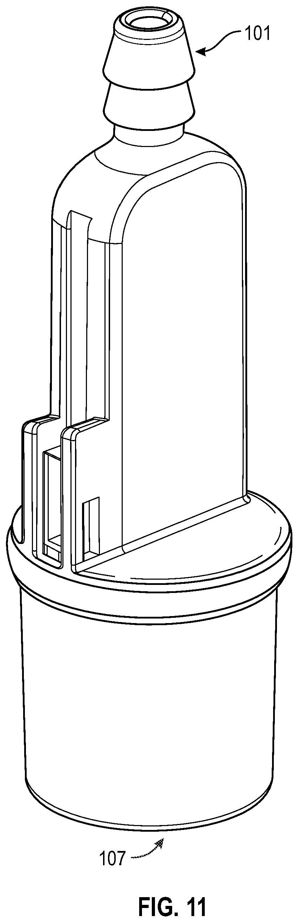

Figure 11 depicts, in accordance with various embodiments herein, an example of a fluidic amplifier.

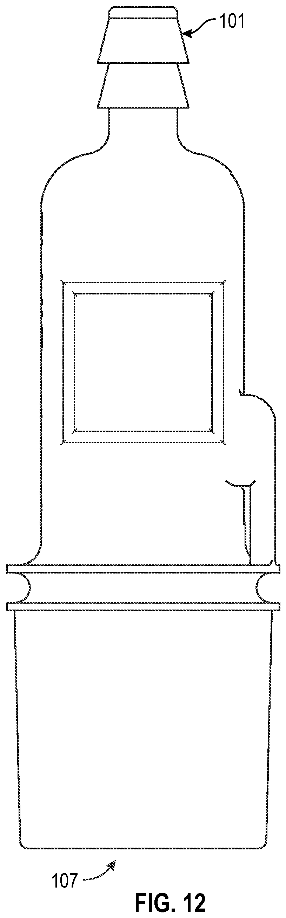

Figure 12 depicts, in accordance with various embodiments herein, an example of a fluidic amplifier.

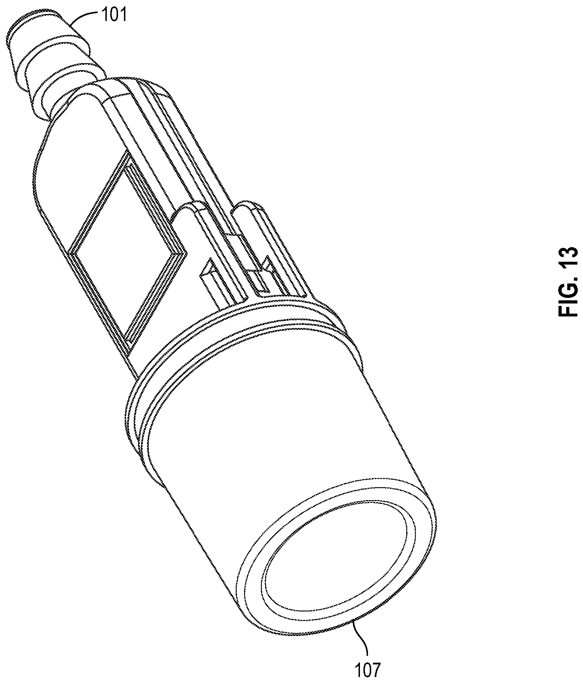

Figure 13 depicts, in accordance with various embodiments herein, an example of a fluidic amplifier.

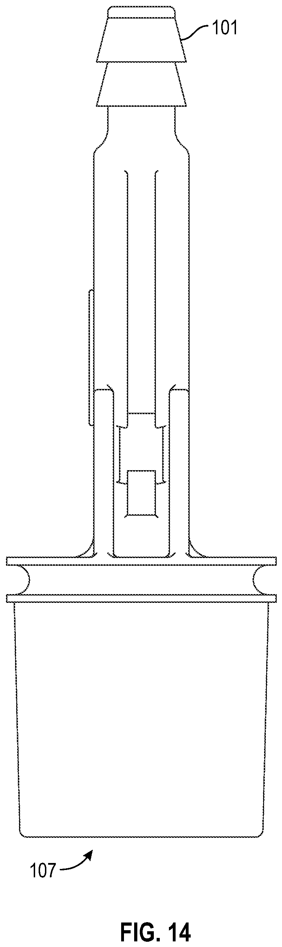

Figure 14 depicts, in accordance with various embodiments herein, an example of a fluidic amplifier.

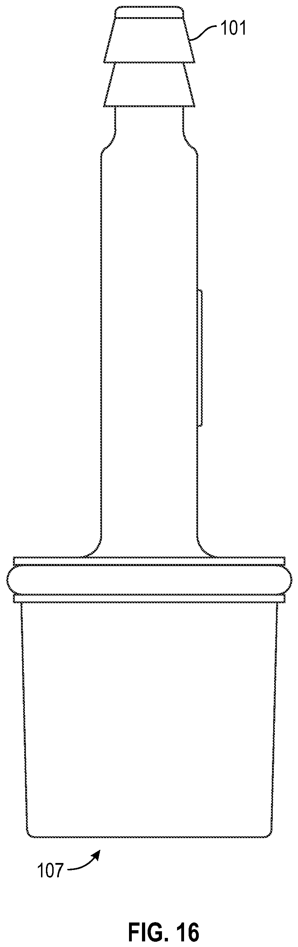

Figure 15 depicts, in accordance with various embodiments herein, an example of a fluidic amplifier.

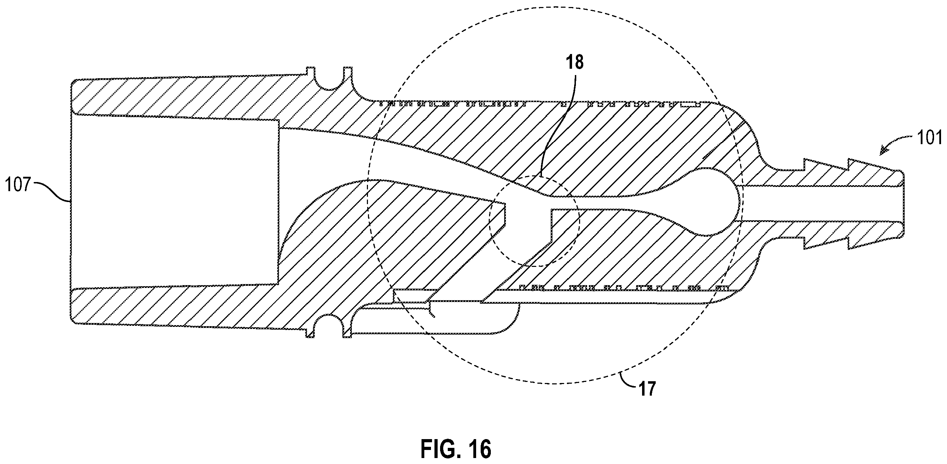

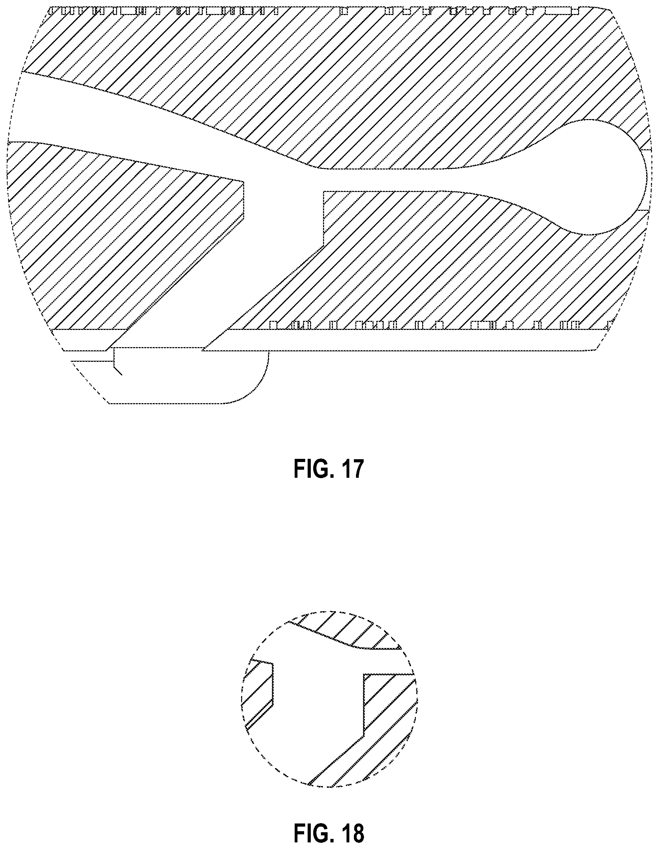

Figure 16 depicts, in accordance with various embodiments herein, an example of a fluidic amplifier. The dotted circle area referenced as 17 is depicted in greater detail in Figure 17 herein. The dotted circle area referenced as 18 is depicted in greater detail in Figure 18 herein.
Figure 17 depicts, in accordance with various embodiments herein, an example of a fluidic amplifier. Figure 17 depicts an area in greater detail that is also referenced in Figure 16 herein.
Figure 18 depicts, in accordance with various embodiments herein, an example of a fluidic amplifier. Figure 18 depicts an area in greater detail that is also referenced in Figure 16 herein.

### DETAILED DESCRIPTION

All references, publications, and patents cited herein are incorporated by reference in their entirety as though they are fully set forth. Unless defined otherwise, technical and scientific terms used herein have the same meaning as commonly understood by one of ordinary skill in the art to which this invention belongs. Kleinstreuer, et al., Modern Fluid Dynamics: Basic Theory and Selected Applications in Macro- and Micro-Fluidics (Springer, 2009 Edition); Hornyak, et al., Introduction to Nanoscience and Nanotechnology, CRC Press (2008); provide one skilled in the art with a general guide to many of the terms used in the present application. One skilled in the art will recognize many methods and materials similar or equivalent to those described herein, which could be used in the practice of the present invention. Indeed, the present invention is in no way limited to the methods and materials described.

As used herein, the terms "coaxial," "in-line," and "coaxially aligned," means to two or more components generally oriented or positioned as having a common axis. In accordance with various embodiments herein, for example, a device with coaxially aligned components may have a fluidic amplifier with one or more coaxially aligned components in one channel, such as for an inspiration phase of ventilation, and one or more coaxially aligned components in another channel, such as for an expiration phase of ventilation. In one embodiment, a fluidic amplifier with one or more coaxial components is a monostable fluidic amplifier. In another embodiment, a fluidic amplifier with one or more coaxial components is a bistable fluidic amplifier. Further, as used herein, "coaxially aligned" refers to a general, rather than precise, orientation or position of a common axis. The present invention is in no way limited to only a precise coaxial alignment.

As used herein, "ventilator" includes ventilator, resuscitator, and respirator. As readily apparent to one of skill in the art, various terms related to ventilation have evolved in the related fields over time and the term "ventilator" may sometimes be used interchangeably to refer to a number of devices related to ventilation. The term "ventilator" as used herein is not intended to limit the invention to only a device that is narrowly defined as a literal ventilator.

As used herein, the abbreviation "BVM" means Bag Valve Mask.

As used herein, the abbreviation "PIP" means Peak Inspiratory Pressure.

As used herein, the abbreviation "PEEP" means Positive End Expiratory Pressure.

As used herein, the abbreviation "RR" means Respiratory Rate.

As used herein, the abbreviation "IE" means Inhale to Exhale Ratio.

As used herein, the abbreviation "CPAP" means Continuous Positive Airway Pressure.

As used herein, "laminar air flow" refers to a process where air is manipulated to force air to move at the same speed and in the same direction, with no or minimal cross-over of air streams in a given space. Similarly, "laminar air flow design" may refer to, for example, an apparatus that contains one or more channels that are designed so that there is no or minimal cross-over of air streams in a given space.

As used herein, "inspiration" refers to the process of taking in air during respiration.

As used herein, "expiration" refers to the process of breathing out air during respiration.

As used herein, "treatment" or "treating" should be understood to include any indicia of success in the treatment, alleviation or amelioration of an injury, pathology or condition. This may include parameters such as abatement, remission, diminishing of symptoms, slowing in the rate of degeneration or decline, making the final point of degeneration less debilitating; improving a patient's physical or mental well-being; or, in some situations, preventing the onset of disease.

As used herein, "diagnose" or "diagnosis" refers to determining the nature or the identity of a condition or disease. A diagnosis may be accompanied by a determination as to the severity of the disease.

As used herein, "prognostic" or "prognosis" refers to predicting the outcome or prognosis of a disease.

As used herein, the term "normal subject" refers to a population when taken as a whole or average, with the average amount of incidence.

As used herein, the terms "fluid amplifier" or "amplifier" or "fluidic amplifier" can refer to any change in the magnitude or vector of the fluid. It is possible, for example, for an "amplifier" to deliver values a fraction of the original input, equal to the original input, or greater than the original input in magnitude, and/or vector.

As described herein, various embodiments relate to the field of ventilation and fluidic amplification. As readily apparent to one of skill in the art, fluidic amplification may be useful for numerous purposes in addition to the medical device or ventilator industry and may be used in conjunction with a variety of purposes across various technical sectors, and is in no way intended to be limited to only use as a medical device or ventilator.

Similarly, as apparent to one of skill in the art, fluidic amplification may be useful to a variety of professionals and environments, and the application is in no way intended to be limited to ventilation in a standard hospital setting, or the specific ventilation or respiratory conditions and settings listed herein. For example, various embodiments herein comprise a device that may be used by cardiopulmonary professionals. Or, for example, various embodiments herein comprise a device that enables emergency ventilation and/or resuscitation by first responders. Similarly, in another embodiment, the device enables ventilation in austere environments. In another embodiment, the device may be used by design engineers in machine design, process controls and power, and/or safety devices. Similarly, as readily apparent to one of skill in the art, various embodiments herein may be used in conjunction with, and/or incorporated into, CPAP (Continuous Positive Airway Pressure) Therapy.

As disclosed herein, the COVID-19 coronavirus pandemic has highlighted a need for and an absence of ventilators and resuscitators that are designed to be quickly manufactured and employed where needed, while still having the capacity to operate effectively. Some of the challenges were the result of a lack of modularization of certain geometries in the design of ventilators and relevant device components, as well as the absence of the ability to timely and efficiently manufacture a customized ventilator or relevant component for a particular patient, including at the point of care. In one embodiment, a device for providing ventilation comprising modular and/or coaxial components. In another embodiment, the device comprises a fluidic amplifier. In another embodiment, the device can be scaled up or down in size and geometry in order to meet desired ventilatory parameters. As readily apparent to one of skill in the art, although various examples of color coded fluidic amplifiers are described herein, the invention is in no way intended to be limited to devices that are constrained to examples of these particular geometries, sizes and dimensions, and may be varied depending on the desired ventilatory parameters. For example, in accordance with various embodiments herein, the device may be scaled down and operate in the capacity of a microfluidic device. Or, for example, the device may be scaled to operate in the capacity of a gigafluidic device. Similarly, as readily apparent to one of skill in the art, although various examples of color coded fluidic amplifiers are described herein, the invention is in no way intended to be limited to devices that are constrained to examples of these particular geometries, sizes and dimensions, and may be varied in the interests of providing modularization and greater manufacturing efficiencies. For example, the device may adapt and adopt certain geometries and dimensions in the interest of more easily interfacing with existing commercially available accessories in the medical field. Or, for example, the device may adopt certain geometries and dimensions in the interest of having the capacity to be quickly manufactured and employed where needed by healthcare professionals in a pandemic, such as by having modularization of certain geometries in the design of ventilators and relevant device components.

In one embodiment, a ventilation device in conjunction with a protocol leveraging respiratory rate, allowing clinicians to quickly assess respiratory mechanics without complex monitors or resources. In another embodiment, the protocol provides a wide variety of pressure profiles.

In one embodiment, a device comprising a fluidic amplifier with one or more coaxially aligned components. In another embodiment, the device functions with no internal moving components. In another embodiment, the device provides a basic, robust fluidic amplifier that can function with no internal moving components that fits in-line of the system's connecting piping/tubing. In another embodiment, the device is a coaxial fluidic amplifier. In another embodiment, the fluidic amplifier provides air ventilation to a user. In another embodiment, the device is part of an overall treatment regimen for subject after infection by the coronavirus Covid-19. In one embodiment, the device is 3-D printed. In another embodiment, the device is injection molded and/or milled.

In another embodiment, a fluidic amplifier that is integrated and/or capable of connecting to a flow generating device. The flow generating device may be manually operated, such as for example, a Bag Valve Mask ("BVM"). In another embodiment, the fluidic amplifier is incorporated into the manually operated flow generating device itself. In accordance with various embodiments herein, the fluidic amplifier may be used to enhance an existing pressure safety system to increase safety to the user.

In one embodiment, the device comprises a monostable fluidic amplifier. In another embodiment, the device comprises a bistable fluidic amplifier.

In another embodiment, a device comprising a ventilator with one or more coaxially aligned components. In another embodiment, the device is for use as an in-line coaxial mechanical ventilator. In another embodiment, the device is used as a mechanical actuator in industrial and/or robotics applications. In another embodiment, the device is used as a component of a medical device. In another embodiment, the device is used as a component of a blood pump. In another embodiment, the device is used as a component in a gas flow monitoring device. In another embodiment, the device is used as a component in a power generating device. In another embodiment, the device is used as a component in a pressure relief and/or pressure maintaining device. In another embodiment, the device is used in an MRI environment.

In accordance with various embodiments herein, the device may be manufactured by one or more techniques, including but in no way limited to, machining, casting, additive manufacturing (3D printing), vacuum forming and/or injection molding.

In one embodiment, a device comprising a fluidic amplifier with one or more coaxially aligned components, wherein the device is adapted to be simple and robust enough to be manufactured and deployed as a disposable mechanical ventilator.

In one embodiment, the device may be modified and/or adjusted by one or more geometries to achieve a desired set of parameters. In another embodiment, the one or more geometries include one or more of the following: the width of Nozzle **102,** depth of Channel **108,** radius of Biased Port Attachment Surface **103,** shape of non-biased outlet shape, divergence angle between biased channel and non-biased channel, and Aero Offset **109** between Nozzle **102** exit and start of radius. In another embodiment, the shape of the nozzle and various channels are geometries that can be adjusted to achieve desired parameters.

In one embodiment, a fluidic amplifier comprising one or more coaxial components, wherein the one or more coaxial components have dimensions that are adapted to provide a desired Inhale to Exhale Ratio (IE) for ventilation by a subject. In another embodiment, dimensions that are adapted include one or more of the following: nozzle **102** width, nozzle **102** depth, radius of edges, channel **108** angle, channel **108** depth, and channel **108** width. In another embodiment, the IE is further modified by gas flow rate. In another embodiment, the IE is further modified by properties of the fluid.

In one embodiment, a device comprising a fluidic amplifier with a plurality of coaxially aligned components. In another embodiment, the plurality of coaxially aligned components comprise one or more of the following: Fluid Inlet **101,** Nozzle **102,** Biased Port Attachment Surface **103,** Non-biased Port-Attachment Surface **104,** Exhaust **105,** Splitter **106,** Outlet **107,** Channel **108,** and Aero Offset **109.** In another embodiment, the fluidic amplifier utilizes laminar air flow design. In another embodiment, the fluidic amplifier is adapted to provide emergency mechanical ventilation. In another embodiment, the device is disposable. In another embodiment, the components are modular in design. In another embodiment, the device may be modified and/or adjusted by one or more geometries to achieve a desired set of parameters. In another embodiment, the device may be modified to achieve a desired set of parameters by one or more of the following geometries: width of Nozzle **102,** depth of Channel **108,** radius of Biased Port Attachment Surface **103,** shape of non-biased outlet, divergence angle between biased channel **108** and non-biased channel **108,** and Aero Offset **109** between Nozzle **102** exit and start of radius. In another embodiment, the desired set of parameters are determined by one or more of the following patient measurements: peak inspiratory pressure (PIP), positive end expiratory pressure (PEEP), and respiratory rate (RR).

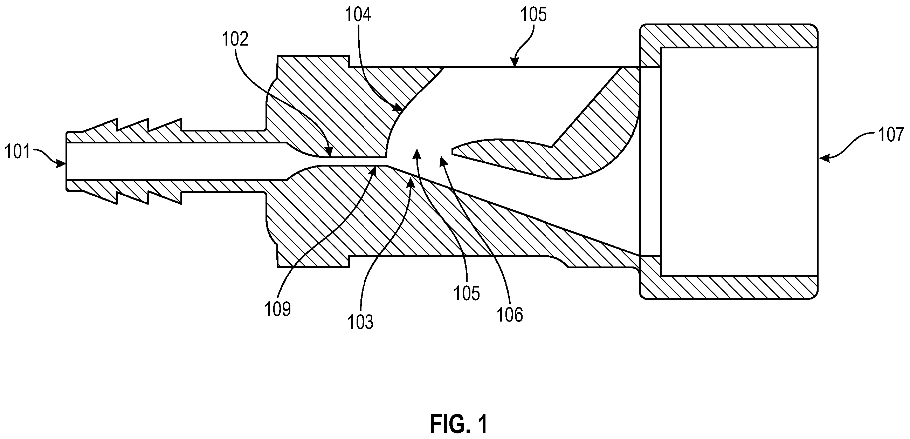

Turning to Figure 1 herein, an example is provided of a device that may be used as a ventilator or resuscitator. In one embodiment, the device comprises a ventilator as described in Figure 1 herein. In another embodiment, the device comprises a ventilator comprising one or more of the following components: Fluid Inlet **101,** Nozzle **102,** Biased Port Attachment Surface **103,** Non-biased Port-Attachment Surface **104,** Exhaust **105,** Splitter **106,** Outlet **107,** Channel **108,** and Aero Offset **109.** In another embodiment, the Exhaust **105** is a non-biased port. In another embodiment, the Splitter **106** provides channel divergence geometry. In another embodiment, the Outlet **107** is a biased port leading to lungs of a user.

In one embodiment, a device comprising a fluidic amplifier with one or more coaxially aligned components, wherein the one or more coaxially aligned components comprising one or more of the following: Fluid Inlet **101,** Nozzle **102,** Biased Port Attachment Surface **103,** Non-biased Port-Attachment Surface **104,** Exhaust **105,** Splitter **106,** Outlet **107,** Channel **108,** and Aero Offset **109.**

In another embodiment, a device comprising a fluidic amplifier with the ability to modularize one or more geometries to achieve a desired set of parameters. In another embodiment, the device comprises a fluidic amplifier with one or more coaxially aligned components. In another embodiment, the device is one of a discrete set of ventilator devices, wherein devices in the set may be of different size, and/or labeled to identify which device best meets the needs of a patient. In another embodiment, the needs of the patient include measurement of levels of peak inspiratory pressure, positive end exhaust pressure, and/or respiratory rate.

In one embodiment, the fluidic amplifier comprises a Channel **108** with a Channel Depth for controlling volume of gas moving through the device. In another embodiment, the Channel Depth influences Respiratory Rate for a user. In another embodiment, the Channel Depth comprises a tear drop shaped channel for stabilizing incoming oxygen gas. In another embodiment, the Channel Depth comprises an inspiration phase channel and/or an expiration phase channel. In another embodiment, the fluidic amplifier comprises a nozzle width for controlling velocity of gas moving through the device. In another embodiment, the fluidic amplifier ventilates a user by fluidics and pressure capacitance. In another embodiment, the fluidic amplifier provides a Respiratory Rate of between 1 to 10 bpm. In another embodiment, the fluidic amplifier provides a Respiratory Rate of between 1 to 80 bpm. In another embodiment, the fluidic amplifier provides a Respiratory Rate of between 4 to 10 bpm, between 7 to 20 bpm, and/or 20 to 40 bpm. In another embodiment, the fluidic amplifier provides a Respiratory Rate of between 5 to 60 bpm. In another embodiment, the fluidic amplifier provides a Respiratory Rate of between 10 to 40 bpm. In another embodiment, the fluidic amplifier provides a Respiratory Rate of between 15 to 35 bpm.

In another embodiment, a device comprising a fluidic amplifier with one or more coaxially aligned components, wherein the device provides the ability to reliably oscillate within a given pressure range so that a robust design may be enabled. In another embodiment, the device may be incorporated to other designs and/or instruments due to the reduced complication this device has for fluidics oscillation. In another embodiment, the device may be used for emergency use ventilation due to ease of use based on the absence of internal moving parts so that reliability is improved and reduction of mistakes in initial emergency care. In another embodiment, the device is relatively small and conducive to transport so that it has applications in austere environments. In another embodiment, the one or more coaxially aligned components reduces complexity and can help ensure users of the device are less likely to become confused and make a mistake when utilizing the device in a critical care setting. In another embodiment, the device is disposable. In another embodiment, the fluidic amplifier is color coded to correlate with a desired Respiratory Rate. In another embodiment, the fluidic amplifier is coded by shape, such as a Circle, Rectangle, and/or Triangle. In another embodiment, the fluidic amplifier is coded by both shape and color.

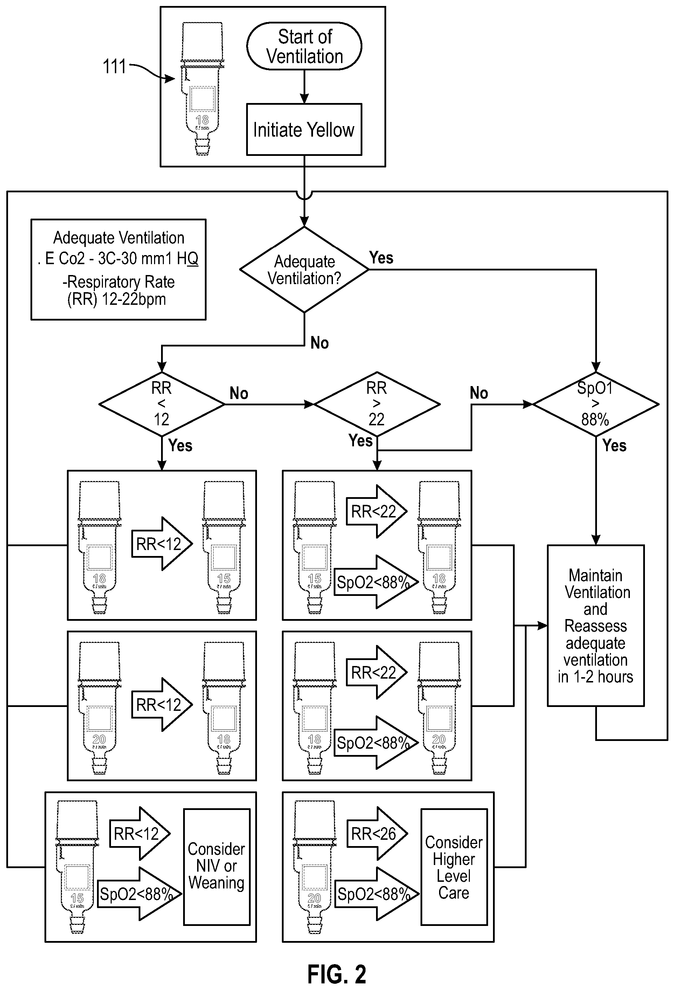

In one embodiment, a method of treating a patient with an adverse condition, comprising providing a device comprising a fluidic amplifier with one or more coaxially aligned components, and ventilating the patient with the device. In another embodiment, the fluidic amplifier is a ventilator. In another embodiment, the device comprises one or more of the following components operably linked: Fluid Inlet **101,** Nozzle **102,** Biased Port Attachment Surface **103,** Non-biased Port-Attachment Surface **104,** Exhaust **105,** Splitter **106,** Outlet **107,** Channel **108,** and Aero Offset **109.** In another embodiment, the adverse condition is a respiratory related condition. In another embodiment, the adverse condition is related to inflammation. In another embodiment, the adverse condition is infection by the coronavirus COVID-19. In another embodiment, a method of treating a patient with an adverse condition according to a protocol as described in Figure 2 herein. In another embodiment, the protocol is a process of ventilation and evaluation for a patient using one or more devices comprising a fluidic amplifier with one or more coaxially aligned components.

In another embodiment, the device may be incorporated with Bag Valve Masks (BVMs). In another embodiment, the device comprises jets to achieve a desired effect. In another embodiment, the device further comprises one or more surface finishes and/or coatings to achieve a desired effect.

The present invention is also directed to a kit to provide breathing assistance. The kit is useful for practicing the inventive method of providing ventilation to a patient. The kit is an assemblage of materials or components, including at least one of the inventive compositions. Thus, in some embodiments the kit contains a composition including one or more color coded fluidic amplifiers with varying dimensions and ventilation parameters, as described herein.

The exact nature of the components configured in the inventive kit depends on its intended purpose. For example, some embodiments are configured for the purpose of treating breathing difficulty associated with infection by COVID-19 coronavirus. In one embodiment, the kit is configured particularly for the purpose of treating mammalian subjects. In another embodiment, the kit is configured particularly for the purpose of treating human subjects. In further embodiments, the kit is configured for veterinary applications, treating subjects such as, but not limited to, farm animals, domestic animals, and laboratory animals.

Instructions for use, or a Protocol, may be included in the kit. "Instructions for use" or a "Protocol" typically include a tangible expression describing the technique to be employed in using the components of the kit to effect a desired outcome, such as to treat a subject with breathing difficulty. Optionally, the kit also contains other useful components, such as, breathing devices and components, mask **118,** pressure relief valve **113,** pressure indicator **114,** anti-asphyxiation valve **115,** Filter **116,** tubing **117,** diluents, buffers, pharmaceutically acceptable carriers, syringes, catheters, applicators, pipetting or measuring tools, bandaging materials or other useful paraphernalia as will be readily recognized by those of skill in the art. In another embodiment, the Filter 116 is a heat and moisture exchange HEPA.

The materials or components assembled in the kit can be provided to the practitioner stored in any convenient and suitable ways that preserve their operability and utility. For example the components can be provided at room, refrigerated or frozen temperatures. The components are typically contained in suitable packaging material(s). As employed herein, the phrase "packaging material" refers to one or more physical structures used to house the contents of the kit, such as inventive compositions and the like. The packaging material is constructed by well known methods, preferably to provide a sterile, contaminant-free environment. The packaging materials employed in the kit are those customarily utilized in treating a subject for breathing difficulty. As used herein, the term "package" refers to a suitable solid matrix or material such as glass, plastic, paper, foil, and the like, capable of holding the individual kit components. The packaging material generally has an external label which indicates the contents and/or purpose of the kit and/or its components.

In one embodiment, a kit may include three (3) fluidic amplifiers (color coded as red, yellow, and green). In another embodiment, the kit may also include a protocol. Following the protocol, the care provider will connect the appropriate fluidic amplifier to the ventilator circuit. Different ventilation characteristics are achieved. In another embodiment, the kit may also include various accessories such as oxygen tubing, a manometer, an anti-asphyxiation valve and/or a Heat and Moisture Exchange HEPA filter. In another embodiment, the kit may include a interface for the user to operate the device. In another embodiment, the kit may also include a flow regulator to set the flow. In another embodiment, the kit may include a plurality of fluidic amplifiers to select and install/uninstall as required per the protocol. In another embodiment, the kit may have only a singular fluidic amplifier to select and install/uninstall as required per the protocol.

Embodiments of the present disclosure are further described in the following examples. The examples are merely illustrative and do not in any way limit the scope of the invention as claimed.

### EXAMPLES

### Example 1

### Advantages

In one embodiment, the technology relates to the field of fluidic amplifiers and this device's use across various technical sectors. In one embodiment, this device is to provide a basic, robust fluidic amplifier that can function with no internal moving components that fits in-line of the system's connecting piping/tubing. In effect, it is a "coaxial fluidic amplifier".

In another embodiment, the device may be for use as an in-line "coaxial" mechanical ventilator. Other embodiments, for example may include the following:
1. For use as a mechanical actuator in industrial and robotics applications
2. For use in medical devices such as blood pumps
3. For use in gas flow monitoring devices
4. For use in power generating devices
5. For use in pressure relief and/or pressure maintaining devices

In accordance with various embodiments herein, some advantages include:
1. In one embodiment, the geometry of a device may be designed to be manufacturing friendly to multiple manufacturing techniques including machining, casting, additive manufacturing (3D printing), and injection molding.
2. In another embodiment, the device is simple and robust enough to be manufactured and deployed as a disposable mechanical ventilator. In another embodiment, by providing a disposable ventilator this greatly reduces or eliminates the need to have OEM dictated service on equipment (a cost burden with traditional ventilators). In another embodiment, by providing a disposable ventilator, the need for sanitization to eliminate cross contamination between patients is eliminated and integrates well with the single use environment already in place in most medical institutions. In another embodiment, by providing a disposable ventilator, statutory ventilator certification requirements can be reduced or eliminated.
3. In another embodiment, the device has a design robust enough to function with minimal adjustments to achieve a minimum and maximum pressure threshold during the mechanical oscillations.
4. In another embodiment, the ability to have an "in-line ventilator" with the possibility of modularized inserts and/or discrete sizes available to a caregiver is a huge enabler for ventilation techniques. This includes but is not limited to transport ventilation, emergency ventilation, animal ventilation.
5. In accordance with another embodiment, due to its simplicity, its size can be relatively small compared to traditional ventilators. This can lead to improvements in the following areas:
   a. Logistics improvements (easier to deliver this device to the point of use)
   b. Reduced manufacturing costs
   c. The relatively small size of the device is a benefit for transport ventilation (i.e. it is easy to transport the individual on the ventilator and it is easy for ventilation to continue while the patient is undergoing medical evaluation such as an MRI).
   d. The material of construction and simplicity is conducive for use in an MRI environment.

In accordance with various embodiments herein, some additional advantages may include:
1. In one embodiment, the device has a design where adjustments can be made to the geometry to achieve a desired set of parameters. This predictability of performance based on these geometries represent a benefit. Some geometries may include the following:
   a. Nozzle Width
   b. Channel Height
   c. Biased Port Attachment Surface Radius
   d. Non-Biased Outlet Shape
   e. Divergence Angle between the Biased and Non-Biased Channels
   f. Aero Offset between the nozzle exit and the start of the radius
2. In another embodiment, a device may have ease to be additively manufactured so that it enables medical professionals to take requisite patient data and manufacture a customized ventilator for that particular patient. This can be done at or near the point of use enabling caregivers to take advantage of distributed manufacturing to rapidly manufacture custom medical devices.
3. In another embodiment, a device is designed to reliably oscillate at very low pressure differentials and flow rates.
4. In one embodiment, the device has the ability to modularize geometries. Thus, a respiratory professional can have a discrete set of ventilator sizes that are labeled in some fashion to help them identify which coaxial ventilator meets the needs of the patient (based on desired PIP, PEEP, Compliance and RR at a given flow rate). This includes, for example:
   a. PIP: Peak Inspiratory Pressure
   b. PEEP-Positive End Expiratory Pressure
   c. RR-Respiratory Rate
5. In another embodiment, the coaxial "in-line" nature of the device is an improvement that eliminates dead space and reduces complexity when compared to traditional ventilators.
6. In another embodiment, features may be added, such as screws, to provide a means to control restriction of flow through certain channels to achieve desired minimum and maximum pressures at a given flowrate (i.e. PIP and PEEP for mechanical ventilation).
7. In one embodiment, dimensions can be manufactured in a cartridge style that can be swapped out if needed to achieve the desired system characteristics.

Some additional advantages of various embodiments herein include:
1. Predictable oscillation-the ability the device offers to reliably oscillate within a given pressure range enables users to make more robust designs.
2. Reduced Complication-The reduced complication this device has for fluidics oscillation over prior art is an enabler for users to incorporate this economical device into their designs.
3. Ease of Use-For emergency use ventilation the ease of use that this device offers with no internal moving parts improves reliability and reduces the risk of mistakes in initial emergency care.
4. The device is relatively small and conducive to transport. This has applications in austere environments.
5. The "coaxial" nature of the device reduces complexity and can help ensure users of the device are less likely to become confused and make a mistake when utilizing the device in a critical care setting.
6. The device can be made "disposable" which has certain logistics and sanitation benefits.

### Example 2

### Table 1 - Example of a Fluidic Amplifier

As further described herein, Figure 1 describes an example of a fluidic amplifier with the following components: Fluid Inlet **101,** Nozzle **102,** Biased Port Attachment Surface **103,** Non-biased Port-Attachment Surface **104,** Exhaust **105,** Splitter **106,** Outlet **107,** Channel **108,** and Aero Offset **109.** In one embodiment, the device is a coaxial fluidic oscillator functioning as a mechanical ventilator. In conjunction with additional embodiments herein, Table 1 below refers to components similarly referenced by number.

**Table 1.**

| **Ref. No.** | **DESCRIPTION** |
|---|---|
| 101 | The inlet port where the motive fluid is introduced |
| 102 | The nozzle. The nozzle geometry, for example, is useful in developing a proper flow to ensure the proper fluid dynamics in the device (i.e. Coanda effect, etc). One claim of the device is the nozzle not being symmetric to the downstream channels. This geometry ensures a certain minimum pressure during the oscillatory cycle. The offset of this nozzle geometry to the centerline of the device is a useful dimension that biases it to a positive Peak Inspiratory Pressure. Additionally, the bias improves pressure recovery which aids in lowering the required motive flow of fluid. |
| 103 | Biased Port-Attachment Surface. This geometry is useful in determining fluid dynamics including the Peak Inspiratory Pressure and the Positive End Exhaust Pressure. The radius of this surface is useful to how the Coanda effect occurs within the device. Once the maximum pressure threshold is achieved the device begins to "switch" to the other port with this Coanda effect attachment on this surface no longer. |
| 104 | Non-Biased Port-Attachment Surface. During the "exhaust" phase of the cycle the fluid begins to attach to this a "bubble" associated with this surface. |
| 105 | The exhaust is where the gas is released outside of the device. The geometry can be made variable to provide adjustability or be a fixed geometry. |
| 106 | Splitter-The geometry of the divergence between the biased and non-biased channels plays a role in the oscillatory motion of the device. |
| 107 | The outlet on the biased port is where the gas travels to during the biased portion of the cycle. As an example-for mechanical ventilators this would be connected to the human lung. Once the pressure capacitance is reached within this outlet the device "switches" to the non-biased port and exhausts. |
| 108 | The channel. The channel depth, for example, is a useful geometry for this device. |
| 109 | Aero Offset is the distance between the nozzle exit and start of the "Biased Port Attachment Surface". This is used to tune Positive End Expiratory Pressure. |
| | Increasing this distance will bias flow to the patient and increase Positive End Expiratory Pressure while decreasing the distance will reduce Positive End Expiratory Pressure |

### Example 3

### Ventilator example

As described herein, the inventors have developed various devices and related methods and components for providing ventilation to a subject. In one embodiment, the ventilator is a single use, constant flow, pressure cycled ventilator designed to provide emergency mechanical ventilation. The ventilator may comprise, for example, a simple fluidic valve, with an inline manometer and factory preset Peak Inspiratory Pressure (PIP) and factory preset Positive End-Expiratory Pressure (PEEP) for the input gas flowrates indicated on the labeling. In accordance with various embodiments herein, it may also incorporate a pressure relief valve (POPOFF) for excessive pressure, and an anti-asphyxiation valve (ASV) to prevent asphyxiation. The ventilator may be connected to the patient by one or more various components, including for example, facemask, Supraglottic Airway, or endotracheal tube. In accordance with various embodiments herein, the ventilator may be a device that is gas powered, fluidic, and load switched valve designed to provide pressure cycling ventilation. Further, the device may have the additional advantage of not having any moving parts, controls, or adjustments, and thus, in accordance with various embodiments herein, come factory set to provide performance indicated on the product labeling.

In one embodiment, a ventilator device is a single use, constant flow, pressure cycled ventilator designed to provide emergency mechanical ventilation, utilizing fluidic characteristics within a fluidic amplifier to oscillate between inspiratory and expiratory phases of ventilation. In accordance with various embodiments herein, there may be three (3) fluidic amplifiers that are changed out as needed, such as may be based on an applicable protocol. These fluidic amplifiers may be denoted, for example, as "red, yellow, or green" amplifiers and referred to within the protocol. In another embodiment, the ventilator device further comprises a pressure indicator, or manometer, with visual indicator of pressure range. In another embodiment, the ventilator device further comprises a pressure relief valve, an anti-asphyxiation valve, a filter and/or gas tubing. In another embodiment, the inlet for the supplied oxygen, such as via the oxygen tubing, is through barbed fitting on the inlet of the fluidic amplifier. In another embodiment, the ventilator device could be used in and in conjunction with a hospital, sub-acute facility, disaster medicine, wilderness medicine, and pre-hospital (EMS) environment and setting, for example.

### Example 4

**Table 2 - An example of a ventilator and specifications**

| **Table 2.** | |
|---|---|
| Indications for Use | In this embodiment, this device is intended to be used by properly trained personnel to deliver emergency, short term, constant flow, pressure cycled, ventilatory support on patients weighing 25kg and above. |
| Patient connection | Face Mask Supraglottic Airway Endotracheal Tube |
| Gas supply required source pressure | 50 psig |
| Maximum Inspiratory Flow | 25L/min |
| Working Mechanism | Exhalation Valve with Load Switched Monostable Fluidic Amplifier |
| Ventilation Frequency | Auto-adjusting to lung capacity |
| Maximum Pressure Relief | 40 cm H₂O |
| Delivered Pressure | Up to 30 cm H2O |
| Delivered Volume | Auto Adjusting to lung capacity |
| Inspiratory Resistance | 3 cm-H₂O/L/sec** |
| Expiratory Resistance | 3 cm-H₂O/L/sec** |
| Peak Inspiratory Pressure (PIP) | 18-30 cm H₂O |
| Positive End-Expiratory Pressure (PEEP) | 6-14 cm H₂O |
| Operational gas flow rate | 0-25 lpm |
| FiO₂ Selector Dial | None |
| Oxygen Concentration | 85% when supplied with 100% O₂ |
| Dead Space of Circuit | < 5mL |
| Reusable | NO |
| Standards | ISO 10651-5 |
| FiO₂ Delivery | FiO₂ of >85% when supplied with 100% O₂ |

| | |
|---|---|
| **As determined by anti-asphyxiation valve | |

In one embodiment, the device is intended to be used by properly trained personnel to deliver emergency, short term, constant flow, pressure cycled, ventilatory support on patients weighing 25kg and above. In another embodiment, the device may be used in a hospital, sub-acute facilities, disaster medicine, wilderness medicine, or pre-hospital (EMS).

### Example 5

### Testing for gas powered resuscitators

Per ISO 10651-5 for gas powered resuscitators, the inventors performed testing for various various embodiments of ventilators, fluidic amplifiers, and other related components and methods further described herein. These tests included:
- Vomitus Resistance
- Water Immersion
- Oxygen Concentration
- Inspiratory Resistance
- Expiratory Resistance
- PEEP Test
- Pressure Limitation
- Drop

The results of these tests demonstrated that various embodiments of ventilators, fluidic amplifiers, and other related components and methods further described herein met performance and design specifications requirements and standards as outlined in requirements and specifications as outlined in ISO 10651-5 - Particular Requirements for Basic Safety and Essential Performance for Gas Powered Resuscitators.

Additionally, an aging test at ~60degC has been completed on various embodiments of the device. There is no noticeable change in performance after 14 days stored at the elevated ~60degC temperature range. Per ASTM F1980 using a Q10 = 2.0 this indicates a 12 month shelf life is an acceptable calculated shelf life.

For applications as a ventilator, the materials may meet the requirements outlined in the ISO 18562 series. Regarding the actual fluidics itself, however, the device is not limited to a specific material required. However, different materials may require different geometries to work correctly due to the differences in the friction factor between the air and various materials (such that affect the fluidic mechanism, or Coanda Effect).

### Example 6

### Table 3 - Fluidic amplifiers coded Red, Yellow, and Green

As further described herein, various embodiments include a device that is a ventilator that is a single use, constant flow, pressure cycled ventilator designed to provide emergency mechanical ventilation. Designed to provide an economical ventilator that can be rapidly produced in large quantities within a relatively short period of time, it may be an analog computer that utilizes gas flow (fluidics) to provide pressure cycled functionality, and has no electronics or software. There is no direct electrical power source utilized by the device. Instead, it relies on fluidics and pressure capacitance to ventilate the patient. Breath Delivery characteristics such as Peak Inspiratory Pressure (PIP), Positive End Expiratory Pressure (PEEP), and Inhale to Exhale Ratio (IE) are functions of the internal geometry of the device and gas flow rate. Since it is a pressure cycled ventilator, Tidal Volumes (TV) and Respiration Rate (RR) are a function of pulmonary compliance and resistance. Since there are no physical controls, breath delivery characteristics are factory specified at the time of manufacture.

In accordance with various embodiments herein, there may be three (3) fluidic amplifiers that may be factory set for breath delivery. In one embodiment, the three (3) fluidic amplifiers are set for breath deliver at the following nominal values in Table 3 below:

**Table 3.**

| Color coded Fluidic Amplifier | INPUT FLOW | PEEP | PIP | IE |
|---|---|---|---|---|
| GREEN | 20 L/min | 6 cm-H2O | 18 cm-H2O | 2.0 |
| YELLOW | 20 L/min | 10 cm-H2O | 24 cm-H2O | 1.8 |
| RED | 20 L/min | 14 cm-H2O | 30 cm-H2O | 1.5 |

### Example 7

### Flow characteristics during inspiration and expiration

As an example, a fluidic amplifier may be connected to a gas source by means of oxygen tubing. The tubing presses over the fluidic amplifier hose barb as well as the DISS hose barb on the outlet upstream flow control valve. The flowrate of the gas is set by the care provider using a flow control valve upstream of the DISS barbed fitting. The flow characteristics are also described in various figures herein.

### Phase 1: Inspiration

Oxygen enters the fluidic amplifier through the barbed fitting on the fluidic amplifier. After the oxygen gas passes through the stabilizing 'tear dropped' shape channel it is directed through a nozzle where its velocity increases. The velocity of the gas, the characteristics of the gas and the geometry of the wall at the exit of the nozzle, cause the gas jet stream to adhere to the 'patient-side' wall and favor the inspiration phase. Oxygen passes through the patient ISO fitting and feeds the patient circuit.

A small percentage of atmospheric air is entrained through the exhaust port during the inspiratory phase due to the geometry and high velocities internal to the device. The patient physiological effects result in the airway pressure increasing thereby causing a decrease in flow rate to the patient. When the flow rate through the patient circuit reaches approximately that of the motive flow rate, the internal energy pressure balance inverts and the flow is rapidly diverted to the exhaust port. The moment of switching to the expiratory phase yields the Peak Inspiratory Pressure (PIP)

### Phase 2: Expiration

The jet stream has now diverted to the exhaust port thereby creating a weak attraction force to the exhaust 'step' of the fluidic amplifier device. This attraction force is one of the key drivers in generating the Positive End-Expiratory Pressure (PEEP). The motive jet stream now results in an active expiration phase where the jet is pulling air out of the patient airway.

Similar to the inspiration phase, when the energy pressure balance inverts, the jet will rapidly flip back to the patient side to start the inspiration phase again.

### Example 8

### Kit components and accessories

In one embodiment, a kit may include three (3) fluidic amplifiers (color coded as red, yellow, and green). In another embodiment, the kit may also include a protocol. Following the protocol, the care provider will connect the appropriate fluidic amplifier to the ventilator circuit. Different ventilation characteristics are achieved. In another embodiment, the kit may also include various accessories such as oxygen tubing, a manometer, an anti-asphyxiation valve and/or a Heat and Moisture Exchange HEPA filter. In another embodiment, the kit may include a interface for the user to operate the device. In another embodiment, the kit may also include a flow regulator to set the flow. In another embodiment, the kit may include a plurality of fluidic amplifiers to select and install/uninstall as required per the protocol.

### Example 9

### Additional studies

Leveraging findings of ventilated patients with COVID-19 associated ARDS, a need was established for having three modules of the ventilator. Level 1 (coded as Green) provided a PIP of 16 cmH₂O and a PEEP of 6 cmH₂O, Level 2 (coded as Yellow) provided a PIP of 22 cmH₂O and a PEEP of 10 cmH₂O, and Level 3 (coded as Red) provided a PIP of 30 cmH₂O and a PEEP of 14 cmH₂O for mild, moderately and restrictive lung disease respectively. Using a lung simulator, three different lung conditions were programmed and exposed each module. V_{T}, flow, pressure and time was measured and each breath summarized.

As a result, all three modules (Red, Yellow, and Green) reached designed P_{AW} targets within 1 cmH₂O. All models provided PEEP levels within 2 cmH₂O and provided minute ventilation values within 1 L/min of target for the compliance level they were designed. The results showed that reliable pressure cycled ventilation is provided. When exposed to COVID-19 ARDS simulated lung conditions, predictable results were confirmed. The use of respiratory rate to determine pressure selection appears a viable option to tidal volume monitoring in disaster, EMS or limited resource environments.

### Example 10

### Principles

Utilizing a more predictable laminar flow range design for a fluidic amplifier, the inventors are able to remove any circuits and simplify the design. For example, in one embodiment, the device developed is rugged, solid-state, has no moving parts and is small enough to place in line with the patient endotracheal tube. In one embodiment, the device provides pneumatically driven, pressure cycled ventilation and is very small (2.2 x 6.3 cm) and lightweight. The ventilator is modular, consists of the fluidic amplifier valve, and a standard medical grade ISO gas source barb connector and a standard patient mask / airway adaptor (15mm/22mm). This device can either assist or control the ventilation of a patient. In one embodiment, it can be customized to target any of the following: PIP of 10-35 cmH₂O, PEEP of 5-20 cmH₂O, I:E ratios of 1:1 -1:3, RR of 5-60 bpm for small or large subjects with healthy or diseased lungs. The fluidic amplifier connected to a compressed air/oxygen source providing a continuous flow of 25 L/min via simple low pressure oxygen tubing provides the pressure-cycling function.

In one embodiment, the basic part of the device comprises a 3D printed device with fluid amplifier channels. Medical grade breathing gases are supplied to the fluid amplifier through the barb port. As the gases flow through the power nozzle of the fluid amplifier, the resulting jet attaches to the right-side wall and into the patient's lungs increasing the pressure. Additionally, there is a small amount of entrainment that comes into the device during inspiration. Once the pressure is reached the geometry is designed for the feedback flow to become great enough to switch the power jets from right to left. This terminates inspiration of the respiratory cycle. The pressure at which this switch occurs depends on the setting of the geometry of the right-side channel. Inspiratory time is a function of how fast the lung fills for the given set pressure. The less time it takes to reach the inspiratory pressure, the higher the elastance of the lung.

When the jet has switched to the left wall, the gases out of the right (from the patient) channel flow until the pressure reaches the set PEEP level. PEEP is set by the geometry of the left channel. The lower the flow rate coming from the patients lungs the faster the PEEP level is reached. Once the set PEEP is reached the feedback flow becomes great enough to switch the device back into inspiration.

Total respiratory system compliance (CRS) influences respiratory rate with a lower respiratory system compliance resulting in a higher respiratory rate. Based on set pressures, known I:E ratios produced (For example, in one embodiment, coded Green and Yellow T₁ estimated at 33% and Red estimated at 36%) and the fixed inspiratory flow rate an estimate of tidal volume and compliance can be determined from the RR.

### Example 11

### Geometry development

Fluidics is a way to process information through a fluid medium as well as transmitting power. As such, one may conceptualize a device as, for example, a preprogrammed black box that will render an output (PEEP, PIP, RR, TV) once its geometrical characteristics (or *features* in Machine Learning terminology) are fixed inside the device.
- Channel Depth - controls the volume of gas moving through the fluidic amplifier, influencing Respiration Rate (RR)
- Nozzle width - controls gas velocity, affecting output pressures, in particular PEEP.

Also, one aim was to develop a predictive model to program the device to perform within the required characteristics of each "color" coded of the family.

### Example 12

### Simulating models and statistical analysis

A basic, supervised regression ML algorithm was used. To solve this task, the learning algorithm is asked to output a function. $f : {ℝ}^{n} \to ℝ$

To predict what features would render the desired output characteristic for the fluidic amplifier.

In order to generate training data, more than 300 devices were 3D printed and bench tested, each one with slight variations in their features (e.g. all fixed but channel depth varying by 0.25 mm each one). With this data, it was possible to generate a prediction model that within a few iterations find the values for the parameters to meet required features (PIP, PEEP, I:E ratio, for example). 3D printing high volumes of devices with inexpensive printers made it possible to generate a high volume of training datasets in a short period of time. It took on average 3 hours per data point per device to complete.

To evaluate ventilators and determine performance capabilities, one could choose to focus on switching pressures (PIP and PEEP), respiratory rate, tidal (V_{T}) and minute volumes (V_{E}). These various parameters could be used to estimate COVID-19 lung conditions, for example. Using the ASL 5000 (IngMar Medical, Pittsburgh, PA) lung simulator with RespiSim System, three different lung conditions (mild, moderate and severe) where programmed, and exposed each model for 50 breaths each (total of minimum of 150 breaths per device) prior to transition to the next set of lung conditions. To simulate ARDS with various respiratory mechanics during controlled ventilation. C_{RS} was varied between 25 and 50 mL/cmH₂O and resistance was varied between 5-15 cmH₂O/L/s to mimic a variety of restrictive lung disease.

The manikin was orally intubated with a 8.0 endotracheal tube with the cuff inflated to 25 mmHg. An inline 14 french suction catheter and HMEF was placed in-line to mimic current COVID-19 recommendations. Tidal volume, flow, pressure and time was measured at 256 Hz. Each breath was summarized by model and lung condition.

Statistical analysis was made utilizing pressure switch settings, anticipated minute ventilation by model and targeted severity of ARDS. Pressure and flow recordings were analyzed using JMP Pro 14 (SAS Institute, Inc). Breath start and expiratory start volume thresholds were set to 5 mL. From the flow tracings, peak inspiratory flow, respiratory rate, T₁, T_{E}, I:E ratio, and V_{T} were calculated. Peak P_{AW}, PEEP, and driving pressure were derived from the pressure waveforms. Performance characteristics considered undesirable and significant were:
- Pressures (P_{AW} & PEEP) that were > +/- 2 cmH₂O from target
- P_{AW} > 30 cmH₂O
- PEEP > 20 cmH₂O
- Driving Pressure > 15 cmH₂O
- Inspiratory times < 0.6 seconds
- Minute ventilations +/- 1 L/min from target
- RR < 8 and > 35

### Example 13

### Results of additional studies

### Pressure Switching:

2,250 breaths were analyzed. Looking at color coded fluidic amplifiers, Green provided 831, Yellow provided 751 and Red provided 668 breaths without difficulty. The mean P_{AW} for all breaths was 16.3 (± 0.46), 22.4 (± 0.71) and 28.3 cmH₂O (± 0.81) for Green, Yellow and Red respectively. The mean PEEP level for all breaths was 7.0 (± 0.62), 10.9 (± 0.73), and 15.7 cmH₂O (± 0.76) for Green, Yellow and Red respectively. The mean driving pressure for all breaths was 9.3 (± 0.79), 11.6 (± 0.50), and 12.6 (± 1.02) for Green, Yellow and Red respectively. All models achieve their targeted P_{AW} within 1 cmH₂O. All models achieved their desired PEEP levels and driving pressure within 2 cmH₂O. Color coded fluidic amplifiers Green and Yellow achieving their set PEEP levels within 1 cmH₂O.

### Respiratory Rate:

Mean respiratory rate (RR) by device designed lung conditions were 17 (± 1.7), 20 (± 1.1), and 28 bpm (± 3.1) for color coded fluidic amplifiers Green, Yellow and Red, respectively. RR for Green, Yellow and Red exceeded 26 in the severe lung condition model, indicating a switch to a higher pressure device or consultation is indicated. RR for Red in the normal lung condition was < 14 indicating a change to a lower pressure for both male and females due to an excessive tidal volume. RR for Yellow was < 16, indicating a change to a lower InVent pressure for females as the tidal volume exceeds 8 mL/Kg.

### Volumes:

Mean V_{TE} by device designed lung conditions were 430 (± 39.1), 393 (± 27.1) and 284 mL (± 47.8) for color coded fluidic amplifiers Green, Yellow and Red, respectively. Green could not deliver a tidal volume of > 220 mL in the severe lung condition and Red delivered a tidal volume of > 570 mL in the mild lung condition. V_{E} produced in all simulated lung conditions fell short of the targeted V_{E}, but by no more than 1 L/min. The Yellow came the closest to the targeted V_{E} of 8.3 L/m.

### Protocol:

By evaluating the performance during the three lung conditions, RR was able to be used as an indicator of when to change the fluidic amplifier color to produce a safe V_{T} range. By starting with Yellow, would allow for a quick assessment of lung compliance by RR produced. From the data and assessment of generally acceptable tidal volumes of 4-8 mL/Kg PBW of an average male being 71 Kg and an average female being 55 Kg it would appear that a RR between 16-26 for females 14-23 for males, would allow one to stay on Yellow and understand that the estimated V_{T} is likely between 220-440 mL for females and between 280-570 mL for males. If the RR is > targeted range, one can consider increasing the pressures to the Red level of 28/14 (DP of 14) as tidal volume is approaching 4 mL/Kg or less for sex. If the RR is < targeted range, V_{T} is approaching 8 mL/Kg or more for sex and you should lower the pressures to the Green level of 16/6 (DP of 10). Additionally, one may say that for the average US Citizen when RR > or < target the patient is at a real risk of having a low V_{T} and hypoventilation or large V_{T} and volutrauma. Adding other non-invasive monitors such ECG, RR, ETCO₂ and SpO₂ to gain a more comprehensive understanding of ventilation and oxygenation could also be helpful if they are available.

### Example 14

### Low, Moderate and High Ranges

In accordance with various embodiments herein, fluidic amplifiers and associated devices and methods may be designated as, and provide, a low, medium (or, moderate), or high strength. Below provides examples of ranges, as referenced by PIP, PEEP and Drive Pressure indications:
PIP
   Low = PIP 16-18 cmH2O
   Moderate = PIP 22-24 cmH2O
   High = PIP 28-30 cmH2O
PEEP
   Low = PEEP 6-8 cmH2O
   Moderate = 10-12 cmH2O
   High = 14-16 cmH2O
Drive Pressure
   Low = 8-10
   Moderate = 11-13
   High = 14-16

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventor that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. Furthermore, it is to be understood that the invention is solely defined by the appended claims. It will be understood by those within the art that, in general, terms used herein, and especially in the appended claims (*e.g.*, bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (*e.g.,* "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (*e.g.*, the bare recitation of "two recitations," without other modifiers, typically means at least two recitations, or two or more recitations).

Accordingly, the invention is not limited except as by the appended claims.

## Claims

1. A medical ventilator for gas fluid flow oscillation between inspiration and exhaust within a pressure range, comprising:
an inlet port where a gas fluid is introduced;
a nozzle;
an outlet leading the gas fluid to the lungs of a user;
an exhaust;
a splitter providing channel divergence between said outlet and said exhaust;
a first port having a biased port-attachment surface downstream of said nozzle and having a radius there-along to said outlet up to a peak inspiratory pressure (PIP) at said outlet;
a second port having a non-biased port attachment surface, wherein upon PIP at said outlet, the gas fluid flow switches from said first port having the biased port attachment surface to said second port having the non-biased port attachment surface and exhaust.

2. The medical ventilator of claim 1 wherein the radius provides fluid dynamics with a Coanda Effect inspiration flow.

3. The medical ventilator of any preceding claim wherein there is an aero offset between the nozzle and a start of said radius.

4. The medical ventilator of any preceding claim wherein said nozzle and said outlet are coaxial.

5. The medical ventilator of any preceding claim and having no internal moving parts.

6. The medical ventilator of any preceding claim being a disposable single use device without need for sanitation.

7. The medical ventilator of any preceding claim wherein said ventilator is manufactured by 3D printing.

8. The medical ventilator of any preceding claim where said ventilator is conducive for use in a MRI environment.

9. The medical ventilator of any preceding claim wherein said exhaust is variable to provide adjustable geometry thereof.

10. The medical ventilator of any preceding claim wherein said non-biased port attachment surface provides a step away from said nozzle and said biased port-attachment surface.

11. The medical ventilator of any preceding claim wherein said non-biased port attachment surface at said nozzle is generally perpendicular thereto.

12. The medical ventilator of any preceding claim wherein the nozzle has a geometry not symmetric to downstream channels.

13. The medical ventilator of any preceding claim wherein the fluid inlet, nozzle, and outlet are coaxial.

14. The medical ventilator of any preceding claim wherein the second port having a non-biased port attachment surface is downstream of said nozzle.

15. The medical ventilator of any preceding claim wherein the biased port-attachment surface is curved.
